# EUROPEAN PATENT APPLICATION

(11) **EP 2 431 021 A1**
(43) Date of publication of application: **21.03.2012**
(21) Application number: 10177061.8
(22) Date of filing: 16.09.2010
(51) Int. Cl.: A61K 8/35, A61K 8/37, A61K 8/45, A61K 8/81, A61Q 17/04

(54) **Sunscreen composition**

(71) Applicant: Unilever N.V., 3013 AL Rotterdam (NL)
(72) Inventor: The designation of the inventor has not yet been filed
(74) Representative: Acham, Nicholas Clive

(57) **Abstract**

The invention relates to a high SPF sunscreen composition. It is an object of the present invention to provide a high SPF (equal to or greater than 15) photo-protective sunscreen composition without compromising on the desired skin sensorial properties while using relatively low amounts of sunscreen agents thereby keeping costs low.

## Description

### Field of the invention

The invention relates to a high SPF sunscreen composition.

### Background of the invention

Solar radiation includes about 5% ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer. Scientific studies have indicated that exposure to UV-A and UV-B radiation for short period causes reddening of the skin and localized irritation, whereas continued and prolonged exposure can lead to sunburn, melanoma and formation of wrinkles. It is also reported that UV radiation causes significant damage to hair. Therefore, it is desirable to protect the skin and other keratinous substrates of the human body from the harmful effects of both, UV-A and UV-B radiation.

Various cosmetic preparations have been reported for preventing and/or protecting the skin from harmful effects of ultraviolet radiation. Numerous organic sunscreen agents capable of absorbing UV-A rays are reported in the field of cosmetics amongst which a particularly useful sunscreen is of the dibenzoylmethane class. Many UV-B sunscreens are also known and approved for safe use in personal care compositions for protection from UV-B radiation. Many cosmetic manufacturers prefer to include both UV-A and UV-B sunscreens in photoprotective compositions so as to provide protection over the entire range of UV radiation. Sun protection Factor (SPF) is a commonly measured attribute of photoprotective compositions which indicates the protection that the skin gets from exposure to both UV-B and UV-A radiation.

Thus, cosmetic manufacturers try to provide consumers with products having higher and higher SPF. One of the ways of achieving this is to incorporate high levels of UV-A and UV-B sunscreens. One disadvantage of this approach is the high cost associated with incorporation of high levels of sunscreens which are expensive. Further, there are safety and regulatory limitations on the upper limit of incorporation of these sunscreens. Sensory properties are also reported to get affected on incorporation of high levels of sunscreens. Hence, there is a problem of achieving high SPF while keeping the total amount of sunscreens in the compositions relatively low.

Various publications on more effective sunscreen compositions have been reported. One such patent publication is US 2005/0063925 which discloses an oil-in-water photoprotective emulsion containing Gemini surfactants and associative polymers. The Gemini surfactant is used as an emulsifier which has at least one dimeric surfactant comprising two surfactant units, which may be identical or different, each consisting of a hydrophilic head and a hydrophobic tail and connected to each other, via the hydrophilic heads, by means of a spacer group. Further, this composition comprises at least one photoprotective system capable of screening out UV rays, containing at least one mineral nanopigment based on metal oxide, and optionally at least one organic, preferably hydrosoluble or liposoluble UV-A and/or UV-B screening agent, and at least one associative polymer comprising at least one C₈-C₄₀ fatty chain.

JP 2008 162 930 (Shiseido) discloses a oil-in-water type emulsion sunscreening cosmetic comprising (a) an oil-soluble ultraviolet absorber (b) a water-soluble thickener (e.g. acrylic water-soluble polymer), (c) a water-soluble ultraviolet absorber and (d) one or more kinds of hydrophilic nonionic surfactant selected from the group consisting of a fatty acid-PEG-glyceryl-based surfactant, a hydrogenated castor oil-based surfactant and a PEG-PPG alkyl ether-based surfactant.

These publications are targeted to ensuring stability of the formulation while having the desired photoprotection and is not reported to enhance SPF. Any attempt at increasing the SPF has been with the use of high amounts of sunscreen compounds.

Therefore there exists a need for a personal care composition comprising sunscreen agents preferably in low concentrations that are able to provide much higher SPF as compared to known sunscreen compositions comprising such low levels of sunscreen agents. It is desirable, if the enhanced SPF benefit could be achieved through synergistic interaction of commonly used ingredients, thereby giving the desired photoprotection benefits at substantially low costs.

The present inventors have been working on solving this problem and have surprisingly found that cosmetic compositions comprising dibenzoylmethane or its derivative in combination with an oil soluble UV-B sunscreen when incorporated in a sunscreen composition along with a non-ionic surfactant of a select class along with a polymer of a select class, provides the enhanced SPF benefits while maintaining the desired sensorial properties when applied on the substrate of interest.

It is therefore an object of the present invention to obviate at least some drawbacks of the prior art and provide high SPF (equal to or greater than 15) photo-protective sunscreen composition.

Another object of the present invention is to provide high SPF sunscreen composition without compromising on the desired skin sensorial properties.

Yet another object of the present invention is to achieve the above objects using relatively low amounts of sunscreen agents thereby keeping costs low.

### Summary of the invention

According to one aspect of the present invention there is provided a high SPF sunscreen composition comprising less than 8% total organic sunscreens comprising,
a) 0.1 % to 5 % by weight dibenzoylmethane or its derivative;
b) 0.1 to 7 % by weight an oil soluble UV-B organic sunscreen;
c) 0.1 to 5% by weight non-ionic surfactant selected from the class of fatty alcohol ethoxylates with saturated carbon chain and having HLB greater than 15.5 or from the class of fatty alcohol ethoxylates with unsaturated carbon chain with HLB greater than 12;
d) 0.1 to 0.5% of a polymer of the class of acrylate / R-methacrylate copolymer or crosspolymer; or an acrylate / R-alkyl acrylate crosspolymer; and
e) a cosmetically acceptable base.

According to another aspect of the present invention there is provided a use of a composition of the first aspect of the invention for obtaining a SPF higher than 15.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions and wash-off shampoos, conditioners, shower gels, toilet bars,. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

By 'A High SPF sunscreen composition' is meant a composition that has an SPF equal to or higher than 15, preferably more than 20, more preferably higher than 25. It is preferred that high SPF is achieved using total UV-B sunscreens in the range of 0.1 to 6%, preferably from 0.5 to 6%, more preferably 1 to 5%, by weight of the composition. It is an advantage of the present invention that the high SPF values are achieved by using low amount of total organic sunscreens. By low amount of total organic sunscreens is meant that the total amount of organic sunscreens is less then 8%, preferably less than 7%, further more preferably less than 6% by weight of the composition.

The invention provides for a high SPF sunscreen composition comprising, a UV- A sunscreen which is a dibenzoylmethane or its derivative; an oil soluble UV-B organic sunscreen in low amount; appropriate amount of a selective non-ionic surfactant; a selective amount of a specific class of polymer; and a cosmetically acceptable base.

The composition of the invention comprises 0.1 to 5% dibenzoylmethane or its derivative. Preferred dibenzoylmethane derivative are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'- methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. Dibenzoyl methane or its derivative is preferably present in 0.2 to 5%, more preferably 0.4 to 3%, by weight of the composition.

The composition of the invention comprises 0.1 to 7%, preferably from 0.5 to 6%, more preferably 1 to 5%, an oil soluble UV-B organic sunscreen by weight of the composition. The oil soluble UV-B organic sunscreen is preferably selected from the class of cinnamic acid, salicylic acid, diphenyl acrylic acid or derivatives thereof. A few of the preferred oil soluble UV-B sunscreens which are commercially available and useful for inclusion in the composition of the invention are Octisalate^{™} (octyl salicylate), Homosalate^{™} (3,3,5-trimethyleyclohexyl 2-hydroxybenzoate), Neo Heliopan^{™} (a range of organic UV filters including ethylhexyl methoxycinnamate (Neo Heliopan AV) and ethylhexyl salicylate (Neo Heliopan OS)), Octocrylene^{™} (2-ethylhexyl 2-cyano-3,3-diphenyl-2-propenoate) or Parsol MCX^{™} (2-ethylhexyl-4-methoxycinnamate). It is interesting to note that only use of oil soluble UV-B sunscreens in the composition of the present invention provide the enhanced SPF benefits of the invention while water-soluble UV-B sunscreens do not provide the desired benefits. It is preferred that the composition of the invention is substantially free of water soluble organic sunscreens. Water soluble sunscreens may however be incorporated in small amount, preferably less than 2%, further more preferably less than 1%, and optimally absent from the composition of the invention.

An important ingredient that contributes to enhancement of SPF of the sunscreen composition of the invention is a selective class of non-ionic surfactant. The non-ionic surfactant is selected from the class of fatty alcohol ethoxylates with saturated carbon chain and having HLB greater than 15.5 or from the class of fatty alcohol ethoxylates with unsaturated carbon chain with HLB greater than 12. It is observed that use of ionic surfactants or non-ionic surfactants of the fatty alcohol ethoxylate class not meeting the claimed criteria do not provide the desired SPF enhancement. Suitable examples of commercially available non-ionic surfactants for use in the composition of the invention are Brij 35((C₂H₄O)₂₃C₁₂H₂₅OH polyoxyethylene lauryl ether) (a C12E023 compound), Brij 97 ((C₂H₄O)₁₀C₁₈H₃₅OH polyoxyethylene 10 oleyl ether) (unsaturated C18EO12) Brij 700 (polyoxyethylene (100) stearyl ether) (C18E0100) or Brij 99 (polyoxyethylene (20) oleyl ether) (unsaturated C18EO20). The non-ionic surfactant is included in 0.1 to 5%, preferably 0.5 to 4%, by weight of the composition.

The composition of the invention comprises 0.1 to 0.5% of a acrylate/ R- acrylate co-polymer or cross-polymer or an acrylate/ R-alkyl acrylate cross polymer. Suitable polymers in this class include Acrylates/Beheneth-25 Methacrylate Copolymer, a Acrylates/Steareth-20 Methacrylate Copolymer, an Acrylate/Steareth-20 Methacrylate Crosspolymer, or an Acrylates/C10-30 Alkyl Acrylate Crosspolymer. Commercially available polymers useful for inclusion in the composition of the invention are Aculyn^{™} 22, Aculyn^{™} 28, or Aculyn^{™} 88, which are available from Rohm and Haas or Pemulen-TR-2 which is available from Lubrizol. Not wishing to be bound by theory, it is observed that inclusion of the specific non-ionic surfactant meeting the above criterion in the composition of the invention provides the desired SPF enhancement, but adversely affects the sensory properties of the product when applied on the skin. It is only with inclusion of the polymers meeting the above criterion that the sensory properties are attained while maintaining the high SPF values. When polymers, not meeting the above criterion, are used, either the desired sensory properties are not achieved or the SPF enhancement is not attained. The polymer is preferably incorporated in 0.1 to 3%, more preferably from 0.1 to 2% by weight of the composition.

The composition of the invention comprises a cosmetically acceptable base. The cosmetically acceptable bases are such as to have a product in preferably a cream, lotion, gel or emulsion format. A more preferred format is a cream, further more preferably one which has a vanishing cream base. Vanishing cream base is one which comprises 5 to 25% fatty acid and 0.1 to 10% soap. In such creams the fatty acid is preferably substantially a mixture of stearic acid and palmitic acid and the soap is preferably the potassium salt of the fatty acid mixture. The fatty acid in vanishing cream base is often prepared using hysteric acid which is substantially (generally about 90 to 95%) a mixture of 45% stearic acid and 55% palmitic acid. Thus inclusion of hysteric acid and its soap to prepare the vanishing cream base is within the scope of the present invention. It is particularly preferred that the composition comprises higher than 7%, preferably higher than 10%, more preferably higher than 12% fatty acid. It has been observed that use of such high levels of fatty acid also contributes to the high SPF. The cosmetically acceptable base is usually from 10 to 99.9%, preferably from 50 to 99% by weight of the composition. The cosmetically acceptable base preferably includes water. Water is preferably included in 35 to 90%, more preferably 50 to 85%, further more preferably 50 to 80% by weight of the composition.

Other useful sun-protective agents e.g. inorganic sun-blocks may be preferably used in the present invention. These include, for example, zinc oxide, iron oxide, silica, such as fumed silica, or titanium dioxide. The total amount of sun block that is preferably incorporated in the composition according to the invention is from 0.1 to 10% by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

According to another aspect of the present invention there is provided use of a composition of the first aspect of the invention for obtaining SPF higher than 15,. The SPF is preferably higher than 20, more preferably higher than 25. The use is preferably for non-therapeutic benefits.

The invention is now further described by way of the following non-limiting examples.

### Examples

### Examples 1 to 11: Effect of various non-ionic surfactants

Various compositions as shown in table 1 were prepared using different non-ionic surfactants. The difference between the various compositions were in the type of non-ionic surfactant used which are detailed in table 2. The SPF of the various compositions (examples 1-11) were measured and the results are shown in table 2. lnvitro-SPF was measured using the Optometrics 290S instrument model. The substrate used was a 8 cm Transpore tape procured from 3M Company. The sample was applied at 2 mg/cm².

**Table 1**

| Ingredients | wt% |
|---|---|
| Hystearic Acid | 17.00 |
| Parsol MCX^{™} | 2.25 |
| Parsol 1789^{™} | 1.20 |
| Non-ionic surfactant | 2.00 |
| Polymer^{#}, Aculyn 28 ^{™} | 0.40 |
| Niacinamide | 1.25 |
| Glycerine | 1.00 |
| Titanium dioxide | 0.90 |
| Potassium hydroxide | 0.57 |
| Silicone oil | 0.50 |
| Perfume | 0.33 |
| Methyl paraben + propyl paraben | 0.30 |
| Water | To 100 |

| | |
|---|---|
| # The polymer is available as a 20% dispersion in water and the dispersion is added at 2% into the composition. | |

**Table 2**

| Examples | Non-ionic surfactant | HLB of non-ionic surfactant | Carbon chain length of non-ionic surfactant | Number of EO groups on non-ionic surfactant | SPF |
|---|---|---|---|---|---|
| Example 1 | Brij -35 | 16.9 | 12 | 23 | 27.0 |
| Example 2 | Brij -700 | 18.8 | 18 | 100 | 29.0 |
| Example 3 | - | - | - | - | 9.7 |
| Example 4 | Brij -97 | 12.4 | 18 (unsaturated) | 10 | 26.0 |
| Example 5 | Brij-99 | 15.3 | 18 (unsaturated) | 20 | 30.0 |
| Example 6 | Brij -30 | 9.7 | 12 | 4 | 12.6 |
| Example 7 | Brij -56 | 12.9 | 16 | 10 | 13.5 |
| Example 8 | Brij-76 | 12.0 | 18 | 10 | 12.0 |
| Example 9 | Brij -52 | 5.0 | 16 | 2 | 10.0 |
| Example 10 | Brij -72 | 5.0 | 18 | 2 | 12.0 |
| Example 11 | Brij -92 | 4.9 | 18 (unsaturated) | 2 | 12.0 |

The sensory property in terms of skin feel on application to the skin was determined for example 1, 2 , 4 and 5 and this was compared to the skin feel of example 3. The study indicated that comparable skin feel is obtained for the examples 1, 2, 4 and 5 as compared to a commercially available sample (example 3).

The data in table 2 indicates that compositions as per the invention (examples 1, 2, 4 and 5) which meet the selective criteria of the non-ionic surfactant provide for vastly improved SPF, with no compromise on the skin feel, as compared to a composition without a non-ionic surfactant and polymer (example 3) and non-ionic surfactant not meeting the selective criteria (examples 6 to 11).

### Examples 12 to 26: Effect of different polymers

Various compositions as shown in table 3 were prepared using different polymers. The difference between these compositions were in the type and/or concentration of polymers which are shown in table 4. These compositions were applied on the skin by a panel of experts and the summary of the sensory perception is summarized in table 4.

**Table 3**

| Ingredients | wt% |
|---|---|
| Hystearic Acid | 17.00 |
| Parsol MCX^{™} | 2.25 |
| Parsol 1789^{™} | 1.20 |
| Non-ionic surfactant Brij - 35 | As in table 4 |
| Polymer | As in table 4 |
| Niacinamide | 1.25 |
| Glycerine | 1.00 |
| Titanium dioxide | 0.90 |
| Potassium hydroxide | 0.57 |
| Silicone oil | 0.50 |
| Perfume | 0.33 |
| Methyl paraben + propyl paraben | 0.30 |
| Water | To 100 |

**Table 4**

| Examples | Wt% of Brij-35 | Polymer | Type of polymer | Wt% of polymer | Sensory perception |
|---|---|---|---|---|---|
| Example 1 | 2.0 | Aculyn - 28 | HASE | 0.25 | Acceptable - Comparable to example 3 |
| Example 3 | - | - | - | - | Commercially used with accepted sensory properties |
| Example 12 | 2.0 | - | - | - | Unacceptable - Very thin and soft |
| Example 13 | - | Aculyn - 28 | HASE | 0.30 | Unacceptable - harder than example 3 |
| Example 14 | 2.0 | Aculyn - 28 | HASE | 0.20 | Acceptable - Comparable to example 3 |
| Example 15 | 2.0 | Aculyn - 22 | HASE | 0.30 | Acceptable - Comparable to example 3 |
| Example 16 | 2.0 | Aculyn - 88 | HASE | 0.30 | Acceptable - Comparable to example 3 |
| Example 17 | 2.0 | Aculyn - 33 | ASE | 0.45 | Unacceptable - Softer than example 3 |
| Example 18 | 2.0 | Aculyn - 44 | HEUR | 0.50 | Unacceptable - Softer than example 3 |
| Example 19 | 2.0 | Aculyn - 46 | HEUR | 0.30 | Unacceptable - Softer than example 3 |
| Example 20 | 2.0 | Natrasol - 330 | HMHEC | 0.10 | Unacceptable - similar to example 10 |
| Example 21 | 2.0 | Aristoflex | @ | 0.2 | Unacceptable - Softer than |
| | | AVC | | | example 3 |
| Example 22 | 2.0 | Aristoflex HMB | @@ | 0.2 | Unaceptable - Softer than example 3 |
| Example 23 | 2.0 | Xanthan Gum | # | 1.0 | Unacceptable - Thicker and harder than example 3 |
| Example 24 | 2.0 | Micro-crystalline cellulose | ## | 1.0 | Unaceptable - Softer than Example |
| Example 25 | 2.0 | U-15 | && | 1.0 | Unaceptable - Softer than Example |
| Example 26 | 2.0 | Pemulen TR-2 | ^^ | 0.2 | Acceptable - Comparable to Example 3. |

The polymers, in table 4 are commercially available as a dispersion in water but the concentration as given in table 4 is on dry weight basis.

The types of polymers used were as follows:
HASE - Hydrophobically modified alkali soluble emulsion which is an acrylate / R-methacrylate copolymer or crosspolymer.
ASE - Alkali soluble emulsion
HEUR - Hydrophobically modified Ethylene Oxide Urethane
HMHEC - Hydrophobically modified Hydroxy ethyl cellulose
@ - Ammonium Acryloyldimethyltaurate/ VP copolymer
@@ - Ammonium Acryloyldimethyltaurate/ Beheneth - 25 Methacrylate crosspolymer
# - Naturally occurring polysaccharide
## - Made from naturally occurring cellulose
&& - U-15 which is a Poly Vinyl Pyrollidone Copolymer available from Induchem.
^^ - Pemulen TR -2 is an acrylate / C10-30 alkyl acrylate crosspolymer.

The data in table 4 indicates that use of a polymer as per the invention along with the selective non-ionic surfactant (examples 1, 14-16, 26) provides for the desired sensory properties while use of only the polymer alone, or only the surfactant alone or use of polymer outside the invention gives unacceptable sensory properties. (examples 12, 13 and 17 to 25).

### Example 27 to 29: Effect of amount of fatty acid

Various compositions were prepared as shown in table 5. They were then tested for SPF similar to the method used for measuring SPF of example 1. The data is summarized in table 5.

**Table 5**

| Ingredients | Example - 27, wt% | Example - 28, wt% | Example - 29, wt% |
|---|---|---|---|
| Hystearic Acid | 10.00 | 15.00 | 20.00 |
| Parsol MCX^{™} | 2.25 | 2.25 | 2.25 |
| Parsol 1789^{™} | 1.20 | 1.20 | 1.20 |
| Non-ionic surfactant Brij - 35 | 2.00 | 2.00 | 2.00 |
| Polymer, Aculyn 28 | 0.25 | 0.25 | 0.25 |
| Potassium hydroxide | 0.60 | 0.60 | 0.60 |
| Water | To 100 | To 100 | To 100 |
| SPF | 15.0 | 22.0 | 21.0 |

The data in table 5 indicates that use of higher levels of stearic acid provides for the high desirable values of SPF.

The invention thus provides for a high SPF sunscreen composition comprising relatively low amount of sunscreen compounds while having acceptable sensorial properties.

## Claims

1. A high SPF sunscreen composition comprising less than 8% total organic sunscreens, the composition comprising,
a) 0.1 % to 5 % by weight dibenzoylmethane or its derivative;
b) 0.1 to 7 % by weight an oil soluble UV-B organic sunscreen;
c) 0.1 to 5% by weight non-ionic surfactant selected from the class of fatty alcohol ethoxylates with saturated carbon chain and having HLB greater than 15.5 or from the class of fatty alcohol ethoxylates with unsaturated carbon chain with HLB greater than 12;
d) 0.1 to 0.5% of a polymer of the class of acrylate / R-methacrylate copolymer or crosspolymer; or an acrylate / R-alkyl acrylate crosspolymer; and
e) a cosmetically acceptable base,
wherein R is a C₁₀₋₃₀alkyl group.

2. A composition as claimed in claim 1 wherein said non-ionic surfactant selected from the group consisting of Brij 35, Brij 97, Brij 700 and Brij 99.

3. A composition as claimed in claim 1 or 2 wherein said polymer is selected from the group consisting of an Acrylates/Beheneth-25 Methacrylate Copolymer, a Acrylates/Steareth-20 Methacrylate Copolymer, an Acrylate/Steareth-20 Methacrylate Crosspolymer, and an Acrylates/C10-30 Alkyl Acrylate Crosspolymer.

4. A composition as claimed in any one of the preceding claims wherein said oil soluble UV-B organic sunscreen is selected from the class consisting of cinnamic acid, salicylic acid, diphenyl acrylic acid and derivatives thereof.

5. A composition as claimed in claim 4 wherein the oil soluble organic sunscreen is selected from Octisalate^{™}, Homosalate^{™}, NeoHelipan^{™}, Octocrylene^{™} or Parsol MCX^{™}.

6. A composition as claimed in any one of the preceding claims wherein said dibenzoyl methane derivative is 4-tert.-butyl-4'-methoxydibenzoylmethane.

7. A composition as claimed in any one of the preceding claims wherein the cosmetically acceptable base is a cream, lotion, gel or emulsion.

8. A composition as claimed in any one of the preceding claims wherein the cosmetically acceptable base comprises 5 to 25% fatty acid and 0.1 to 10% soap.

9. A composition as claimed in claim 8 comprising at least 7% fatty acid.

10. A composition as claimed in any one of the preceding claims wherein the composition has a SPF equal to or higher than 15.
